# EUROPÄISCHE PATENTSCHRIFT

(11) **EP 0 483 607 B1**
(45) Veröffentlichungstag und Bekanntmachung des Hinweises auf die Patenterteilung: **23.04.1997**
(21) Anmeldenummer: 91117712.9
(22) Anmeldetag: 17.10.1991
(51) Int. Cl.: D01G 31/00, D01G 13/00, G01N 33/36

(54) **Verfahren zum Feststellen einer Eigenschaft eines Faserverbandes**
Method for identifying the quality of a fiber material
Procédé pour identifier la qualité d'un matériau fibreux

(30) Priorität: 02.11.1990 CH 3496/90
(43) Veröffentlichungstag der Anmeldung: 06.05.1992
(73) Patentinhaber: MASCHINENFABRIK RIETER AG, CH-8406 Winterthur (CH)
(72) Erfinder: Faas, Jürg, CH-8474 Dinhard (CH)

(56) Entgegenhaltungen:
- EP-A- 0 362 538
- GB-A- 2 210 907
- US-A- 4 758 968
- US-A- 4 766 647

## Beschreibung

Aus der europäischen Patentanmeldung EP-A-0 362 538 der Anmelderin ist ein Verfahren zum Mischen von Textilfasern von unterschiedlicher Provenienz bekannt, in welchem jede Provenienz vorgegebene Fasereigenschaften aufweist und in welchem jede Provenienz eine Mischkomponente von vorgegebenem prozentualen Anteil ist, sowie in welchem die Mischung als Ganzes vorgegebene Fasereigenschaften aufweist und die Mischkomponenten jederzeit steuerbar variable Komponenten sind. Dabei ist der prozentuale Anteil jeder Komponente, um die genannten Fasereigenschaften der Mischung zu erreichen, unter Berücksichtigung der Fasereigenschaften der einzelnen Komponenten automatisch optimiert, indem die Komponentenmischung in Abhängigkeit von vorgegebenen und festgestellten Eigenschaften eines nachfolgend hergestellten Zwischenproduktes, vorzugsweise eines Kardenbandes oder eines nachfolgend hergestellten Endproduktes, vorzugsweise eines Garnes, bestimmt und bei Abweichungen davon unverzüglich und automatisch korrigiert werden.

Das Verfahren der genannten europäischen Patentanmeldung zeigt und beschreibt die Möglichkeit, die Textilfasern von unterschiedlicher Provenienz entweder direkt von den Faserballen oder aus Komponentenzellen einem Mischer zu übergeben, in welchem diese Komponenten homogen gemischt werden. Das Produkt des Mischers wird anschliessend in einer Putzerei gereinigt und im folgenden zur Verarbeitung einer Karde übergeben. Nach der Karde wird das Kardenband in einem Farbprüfgerät geprüft und das entsprechende Signal einer Steuerung abgegeben, welche die Komponentenmischung steuert.

Unter dem Begriff Provenienz können Baumwollfaserballen oder Faserballen mit synthetischen Fasern verstanden werden, so dass es sich bei der Mischung um Mischungen unterschiedlicher Baumwollarten oder um eine Mischung von Baumwollfasern und künstlich hergestellten Fasern handeln kann.

Dabei besteht die Notwendigkeit, den Anteil an Kunstfasern an einer Fasermischung feststellen zu können, was bisher mittels einer Laboranalyse auf chemischer Basis an einzelnen Proben durchgeführt wurde, was einen entsprechenden Zeitaufwand erfordert. In US-A-4,758,968 ist ein Verfahren und eine Vorrichtung beschrieben zur kontinuierlichen Messung der Veränderungen von textilen Faserlunten. Dazu sollen die Veränderungen von einem oder mehreren Parametern der textilen Faserlunte gemessen werden. Aus den ersten Ausführungsbeispielen geht hervor, dass das Signal von einem Signalwandler, der zumindest einen Parameter, z.B. die lineare Faserdichte, überwacht, in drei verschiedenen signalintegrierenden und signalglättenden Schaltungen mit einer unterschiedlichen Zeitkonstante verarbeitet wird. Daraus lassen sich langzeitige Fehler in einem Bereich von Wellenlängenbändern extrahieren. Aus den weiteren Ausführungsbeispielen (Fig. 9 bis 12) geht ferner hervor, dass auch mehrere Signalwandler verwendet werden können, um die Langzeitveränderungen eines Parameters zu beobachten. Diese Messungen können jedoch nicht dazu verwendet werden, um die Mischungsanteile in einem Faserverband festzustellen.

Mit der jetzigen Erfindung, nach den Ansprüchen 1 und 9, besteht nun die Mköglichkeit, insbesondere die Konzentration an Kunsffasern im Gesamtgemisch feststellen zu können.

Dies geschieht vorteilhafterweise, indem die Messung durch Beleuchten des laufenden Faserverbandes an einer Prozessstelle geschieht, an welcher eine Verdichtung des Faserverbandes durch Zusammenführen oder Pressung stattfindet, wobei an der Messstelle eine Lichtdurchführung so angeordnet wird, dass der Prozessablauf nicht beeinträchtigt wird und durch diese Lichtdurchführung das vom Messgut zur Bandlaufrichtung rückgestrahlte Licht aufgefangen und ausgewertet werden kann. Als Messstelle kann dabei eine Prozessstelle gewählt werden, an der das Faserverband in sich überlagernden Schichten abgelegt wird.

Der Vorteil der Erfindung besteht darin, dass auf relativ einfache Weise in einem früheren Stadium des Faserverarbeitungsprozesses die Mischung an natürlichen und künstlichen Fasern mit genügend hoher Genauigkeit festgestellt werden kann, was insbesondere deshalb von Vorteil ist, weil die künstlichen Fasern meist einen wesentlich anderen Preis aufweisen als die natürlichen Fasern und deshalb ein genaues Einhalten des Kunstfaseranteiles in verschiedenen Ländern gesetzlich vorgeschrieben ist.

Im Folgenden werden anhand einiger Vorrichtungsbeispiele Möglichkeiten dargestellt, um das erfindungsgemässe Verfahren durchzuführen.

Es zeigt:
- Fig. 1a bis 1d: das Verfahren und Verfahrensvarianten schematisch dargestellt,
- Fig. 2a bis 2d: eine beispielhafte Ausführungsform der erfindungsgemässen Vorrichtung für die Verfahrensvariante, die eine prozessbedingte Bandkompression ausnützt,
- Fig. 3a und 3b: eine weitere Ausführungsform für die Verfahrensvariante mit einer speziell für die Messung durchgeführten Bandkompression.
- Fig. 4 und 5a,5b: je eine weitere Ausführungsvariante zur Durchführung des Verfahrens an einer Bandablage.

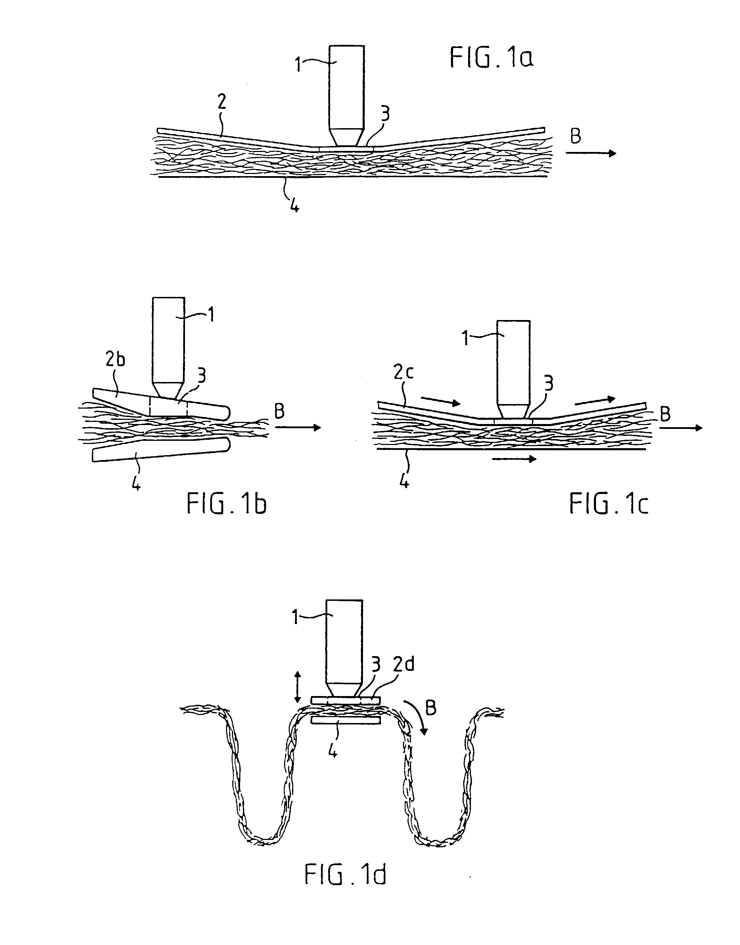

Fig. 1 zeigt eine Messonde 1, welche senkrecht zur Laufrichtung B eines Faserverbandes oder auch Faserbandes angeordnet ist und die Bandoberfläche in konstantem Abstand dazu vorbeigeführt wird. Die Messung könnte auch an einem stillstehenden Faserverband durchgeführt werden. Das Faserband wird, bevor es die Messstelle durchläuft, durch Komprimierungsmittel 2 leicht komprimiert. Die Messung wird durch einen, entsprechend transparent gestalteten, Vorrichtungsteil 3 (Planglas-Scheibe) hindurch gemacht oder durch eine kleine Öffnung (ohne Planglas-Scheibe) zwischen den Komprimierungsmitteln hindurch. Durch entsprechende Ausgestaltung der Komprimierungsmittel, seien sie nun prozessbedingt oder rein messungsbedingt, wird dafür gesorgt, dass an der Messstelle das Band eine genügende Breite hat, um die ganze Messöffnung der Messonde zu decken.

Die Messung der genannten Konzentration durch Auswertung von Spektren wird im nahen Infrarotbereich mit einem FT-NIR-Spektrometer der Firma Bühler, CH-9240 Uzwil, durchgeführt.

Für die Komprimierung des Faserbandes im Gebiet der Messstelle stehen drei Verfahrensvarianten zur Verfügung. Nach Variante 1 (Fig. 1b) wird die Messung an einer Prozessstelle durchgeführt, an der das Faserband prozessbedingt komprimiert wird, wo also das Komprimierungsmittel 2b, z.B. ein Trichter, einen prozesseigenen Vorrichtungsteil darstellt.

Nach Variante 2 (Fig. 1c) wird die Messung an irgendeiner Prozessstelle durchgeführt, und das Faserband wird im Gebiet der Messstelle bremsungsfrei, zwischen mit dem Band mitlaufenden Komprimierungsmitteln 2c komprimiert.

Nach Variante 3 (Fig. 1d) wird die Messung an einer Prozessstelle durchgeführt, an der das Faserband lose hängt (Schlaufen = Speicher), so dass es für die Messung kurz angehalten und zwischen stationären Komprimierungsmitteln 2d komprimiert werden kann.

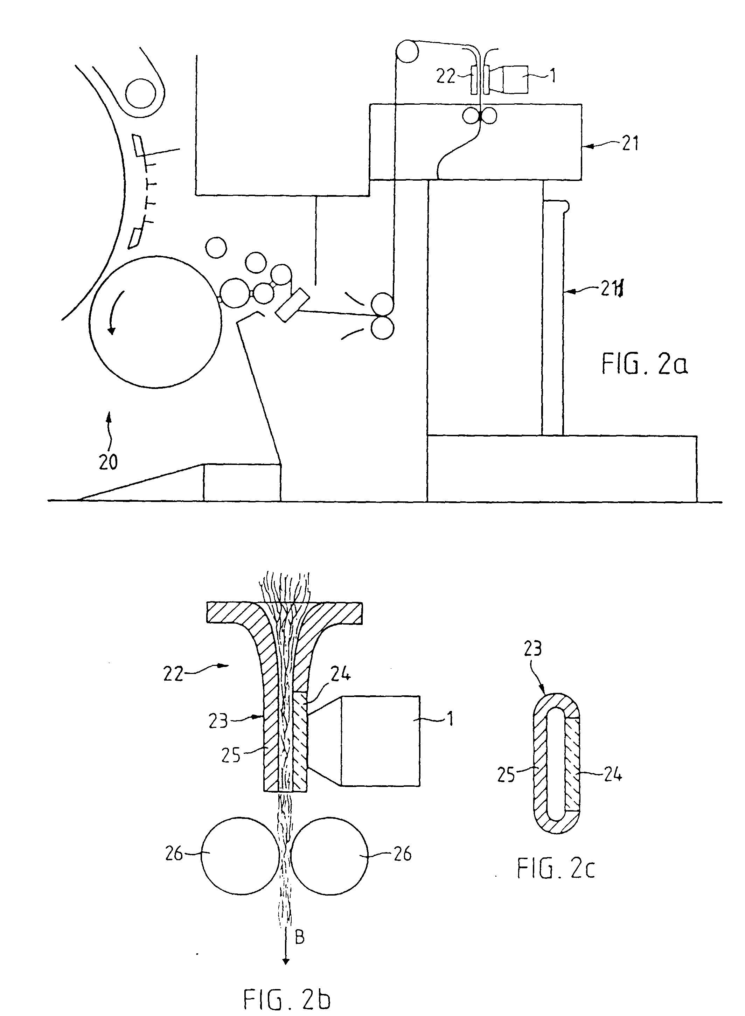

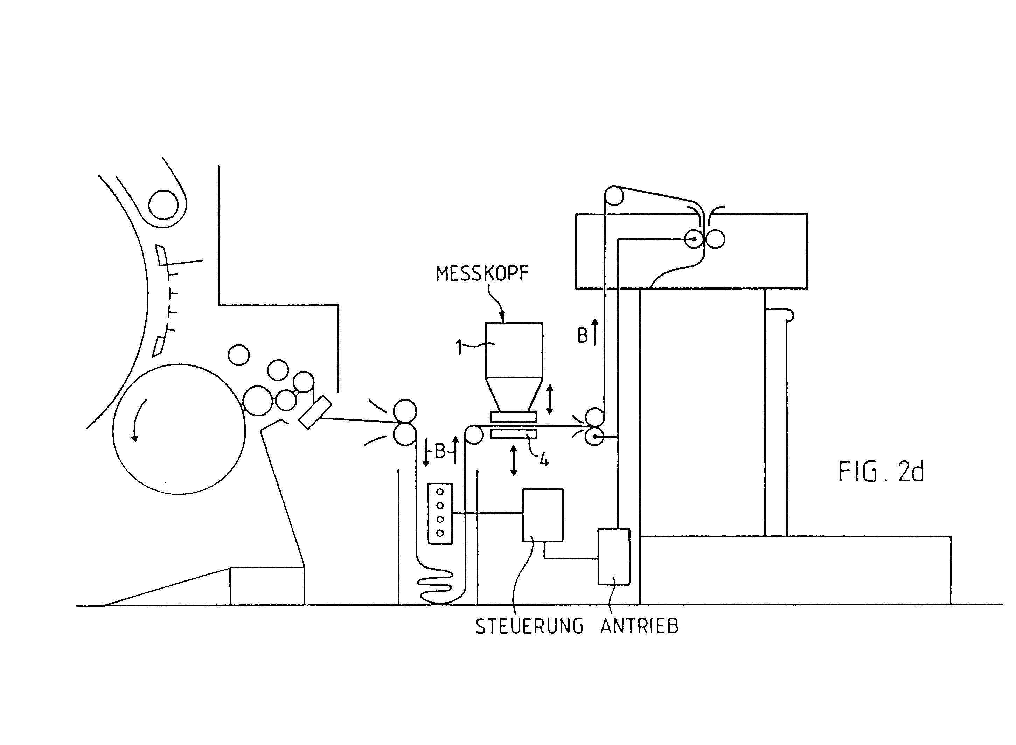

Die Fig. 2 zeigt eine beispielhafte Ausführungsform der erfindungsgemässen Messvorrichtung. Für die Komprimierung des Faserbandes wird eine prozessbedingte Komprimierung in einem Trichter ausgenützt, es wird also die Verfahrensvariante 1 (Fig. 1b) angewandt. Es kann sich dabei wie in Fig. 2a, schematisch dargestellt, um den Trichter einer Bandablagevorrichtung, wie sie für die Bandablage in Kannen benutzt werden, handeln. Es kann sich aber auch um den Zuführtrichter zu einem Stufenwalzenpaar oder um einen Messtrichter, der zur Messung der Banddichte installiert ist, handeln.

Die Fig. 2a zeigt als Beispiel einer Lokalisierung der erfindungsgemässen Vorrichtung schematisch den Austritt des Faserbandes aus einer Karde 20 und seine Zuführung zu einer Bandablagevorrichtung 21 auf einer Kanne 211. Die Messonde 1 ist am Trichter 22 der Bandablagevorrichtung installiert. Der Trichter 22 ist, wie aus Fig. 2b ersichtlich, für diesen Zweck speziell ausgestaltet. Der Trichterhals 23 ist als Messcuvette ausgebildet. Damit das durchlaufende Band eine für die Messung genügende Breite aufweist, hat der Trichterhals 23 den in Fig.2c gezeigten Querschnitt mit der Form eines sehr flachen Rechteckes mit abgerundeten Ecken. Die der Messonde zugewandte breite Seite 24 des Trichterhalses 23 besteht aus einem durchsichtigen Material. Auf den Ablagetrichter folgen in Wandlaufrichtung ein paar Transportrollen 26. Fig. 2d zeigt in Anlehnung an Fig. ld die Steuerung der Schlaufenbildung, die beispielsweise in der DE-B-1931929 beschrieben ist und erfordert in dieser Gestalt ebenfalls Start-Stopp der Kannenfüllung bei der Messung.

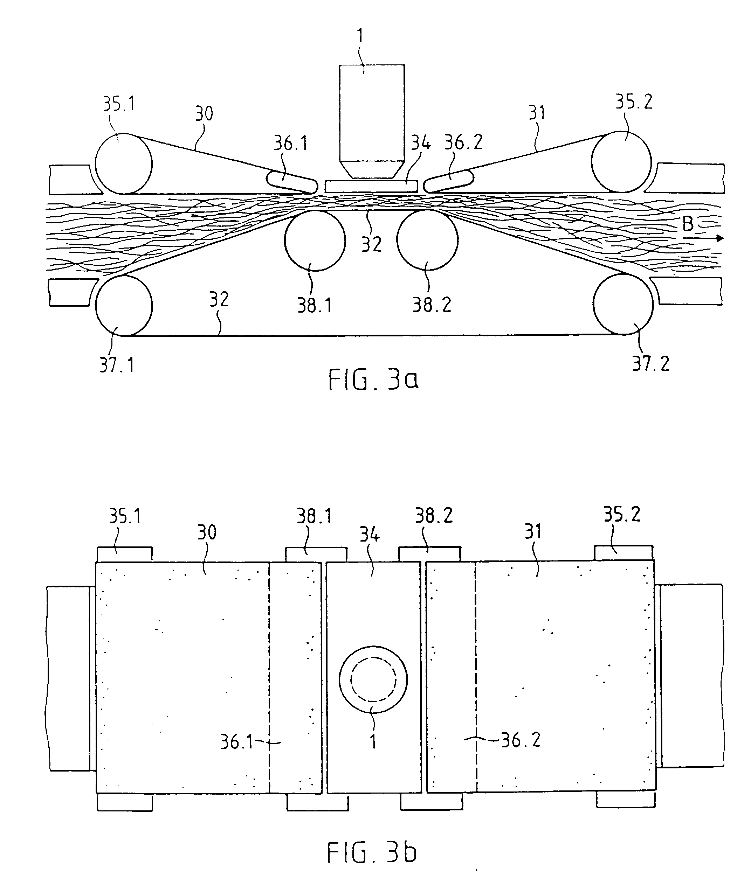

Die Fig. 3 zeigt als Seitenansicht (Fig. 3a) und Draufsicht (Fig. 3b) eine beispielhafte Ausführungsform der erfindungsgemässen Vorrichtung, die für die Durchführung des erfindungsgemässen Verfahrens geeignet ist, und in der das durchlaufende Faserband gemäss der Verfahrensvariante II (Fig. 1c) speziell für die Messung komprimiert wird. Die Vorrichtung kann an irgendeiner Prozessstelle zur Verwendung kommen, an der ein Faserband über eine genügend lange Strecke frei transportiert wird. Da das Faserband durch die Komprimierung nicht gebremst werden darf, muss es durch die Messstrecke aktiv transportiert werden. In der abgebildeten Ausführungsform wird das Faserband beispielsweise durch endlose Riemen 30,31 und 32 transportiert, wobei die Riemen 30 und 31 geradlinig ausgerichtet sind, mit einer zwischen ihnen liegenden durchsichtigen Platte 34 auf der einen Seite, wobei der Riemen 32 auf der andern Seite des Faserbandes derart angeordnet ist, dass zwischen der durchsichtigen Platte 34 und dem Riemen 32 eine verengte Messstrecke gebildet wird. Der Riemen 32 wirkt also gleichzeitig als Komprimierungsmittel. Auf der vom Faserband abgewandte Seite der durchsichtigen Platte ist die Messonde 1 positioniert. Die Platte 43 ist als einziger Teil, der mit dem durchlaufenden Band in Berührung kommt, stationär. Da das Band aber beidseits der Platte durch die Riemen transportiert wird, ist der Bremseffekt vernachlässigbar. Die durchsichtige Platte 34 kann auch wegfallen und die Messung durch eine entsprechende Lücke zwischen den Riemen 30 und 31 durchgeführt werden.

Die beiden Riemen 30 und 31 werden durch die Antriebsrollen 35.1 und 35.2 angetrieben und angrenzend an die durchsichtige Platte 34 um zwei plattenförmige Kulissen 36.1 und 36.2 geführt. Durch Anwendung solcher plattenförmiger Kulissen wird es möglich, einerseits die Riemen bis an die durchsichtige Platte 34 in derselben Ebene zu führen, in der die Platte 34 liegt, andererseits bleibt auf der vom durchlaufenden Faserband abgewandten Seite der Platte 34 Raum, um die Messonde genügend nahe an der Platte 34 zu positionieren. Ähnliche Riemenantriebe sind z.B. bekannt aus der DE-A-3327966, die die Anwendung solcher Riemenantriebe in einem Streckwerk beschreibt.

Der Riemen 32 wird von zwei Antriebsrollen 37.1 und 37.2 angetrieben und über zwei geschleppte Rollen 38.1 und 38.2 geführt. Der Abstand zwischen den Rollen 37 und den Riemen 30 und 31 entspricht etwa der Dicke des der Messstelle zugeführten Bandes. Der Abstand zwischen den Rollen 38 und den Riemen 30 und 31 ist kleiner und entspricht dem für die Farbmessung geeigneten Komprimierungsgrad des Faserbandes.

Damit die Vorrichtung das Faserband in keiner Weise verändert, also im speziellen nicht streckt oder bremst, ist es sehr wichtig, dass die Riemengeschwindigkeiten der Riemen 30, 31 und 32 der Transportgeschwindigkeit des Faserbandes an der entsprechenden Prozessstelle genau entspricht. Ist diese Geschwindigkeit konstant, muss die Geschwindigkeit der Antriebswalzen 35 und 37 entsprechend eingestellt sein. Ist diese Geschwindigkeit nicht konstant, wie z.B. am Ausgang eines Streckwerkes, dessen Streckwalzengeschwindigkeiten über die Banddicke geregelt werden, müssen die Geschwindigkeiten der Antriebswalzen 35 und 37 ebenfalls von dieser Regelung reguliert werden.

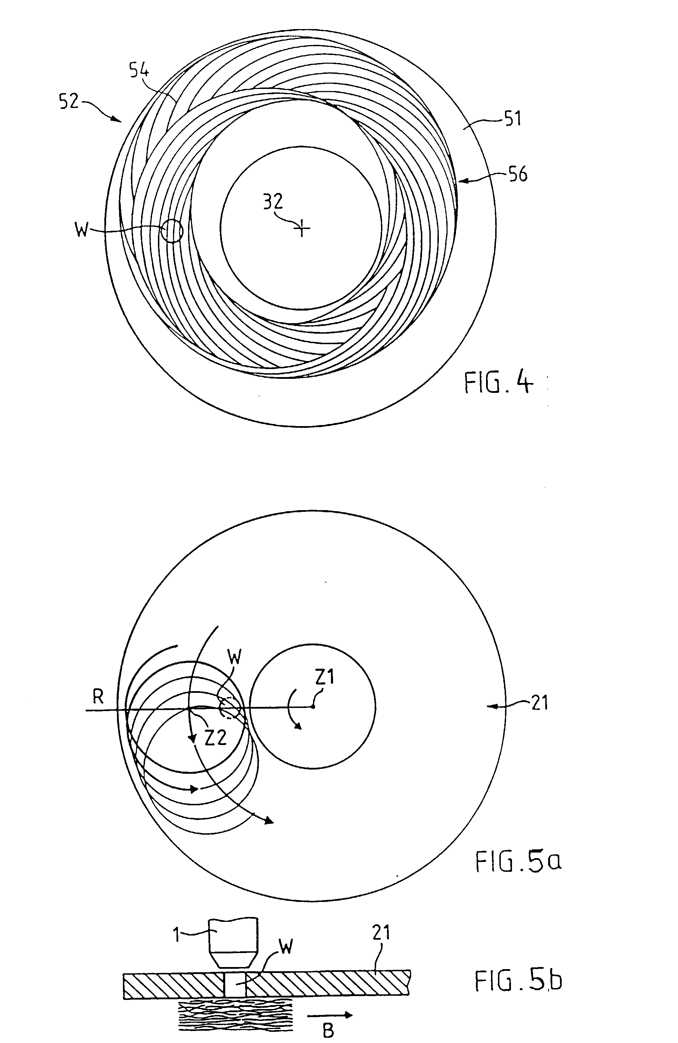

Fig. 4 zeigt schematisch eine andere bevorzugte Messstelle im Bereich der Bandablage, in welchem Bereich sich ein Prozessabschnitt besonders gut für die Messung von Faserbändern eignet. In der Kannenablage wird das Faserband zwischen dem Kannenboden und dem Bandablageoberteil spiralförmig zu einem geordneten "Bandkuchen", das ist eine bestimmte Form einer kontinuierlichen Ablage des Bandes, abgelegt und zusammengepresst. Letzteres, indem ein verschiebbarer Kannenboden das Band von unten gegen den Oberteil drückt, wobei gleichzeitig das Faserband kontinuierlich zwischen Bandkuchen und Band-ablageoberteil eingebracht wird. Zwischen der sich ständig neu bildenden Oberfläche des Bandkuchens und dem Bandablageoberteil findet eine Relativbewegung statt; der Bandkuchen dreht sich um die Kannenachse und beim Ablegen des Faserbandes wird so immer neues Fasermaterial am Bandablageoberteil vorbei bewegt. Hier sind prozessseitig günstige Messbedingungen. Wird nun im Bandablageoberteil eine Mess-Hilfsvorrichtung, beispielsweise eine Messöffnung W angeordnet, durch welches das von unten an den Bandablageoberteil angepresste Fasergut beleuchtet und gemessen werden kann, so ist es möglich, das sich ständig selbst für die Messung vorbereitende und selbsterneuernde Fasergut ohne jeglichen Eingriff in den Prozessablauf zu messen. Das fortlaufend abgelegte Faserband wird kurz nach dem Ablegen am Sensor vorbeigeführt und kann von diesem gemessen werden. Das Messfenster ist an der Schnittstelle mit dem Messgut selbstreinigend und sensorseitig kann eine nicht dargestellte Reinigungs- und Eichvorrichtung angeordnet werden. Damit wird die Messöffnung im Bandablageoberteil entweder kontinuierlich von Staub befreit, beispielsweise indem ein Luftstrahl daran vorbei geführt wird, oder diskontinuierlich mit einem mechanischen Reinigungswerkzeug, beispielsweise einer Bürste. Die diskontinuierliche, mechanische Reinigung der Messöffnung kann wie die Kalibrierung entweder off-line und manuell durchgeführt werden oder automatisch gesteuert oder geregelt. Die Vorteile dieser Mess-Stelle sind einerseits, dass das Messgut "undurchsichtig" ist, das heisst, die Schichtdicke und Schichtdichte des Fasermaterials ist so gross, dass der Hin-tergrundeffekt wegfällt, und dass die Fasern des Messgutes (nach der Karde oder Strecken) gereinigt und geordnet (parallelisiert) und in regelmässiger Anordnung (als Bandkuchen) gespeichert vorliegen, weswegen die Messung von zeitlich wechselnden Störeinflüssen verschont bleibt. Einzig zu beachten ist, dass das Faserband nicht in Richtung seiner natürlichen Längsorientierung am Sensor vorbeigeführt wird, so dass Helligkeitsschwankungen auftreten. Diesem Einfluss kann man entgegenwirken, indem man einen in Prozessrichtung vorgelagerten Hilfs-Sensor zur Bestimmung der Banddichte, beispielsweise einen kapazitiven Sensor, verwendet und mit diesem die Farbmessung für den Messvorgang auslöst.

Der hier bevorzugte Messort im Prozessablauf ist, wie schon gesagt, auf der Bandablage, genauer im Bandablageoberteil. In Fig. 4 ist eine bestimmte, auf den Prozess abgestimmte Beobachtungsstelle für das Messgut in Form einer Messöffnung W angeordnet, welche sich für eine Farbmessung der vorgeschlagenen Art eignet. Eine Kanne 52 zeigt auf dem Kannenboden 51 liegende Bandkuchenlagen 56 eines Faserbandes 54. Diese exzentrisch um den Kannendrehpunkt 32 gelegte Lage wird beim Auffüllen der Kanne durch weitere, in Drehrichtung exzentrisch verschobene Lagen überdeckt, so dass der Kannenboden nach wenigen Bandkuchenlagen gänzlich überdeckt ist. Dies ist in der Regel nach weniger als einer Minute Füllzeit der Fall. Die Oberfläche des Bandkuchenstapels besteht dann aus einer makroskopisch komplizierten Ordnung von Faserbandverläufen, die aber, durch die Messöffnung betrachtet, wegen ihrer Gesamtbewegung um ein Drehzentrum stets den gleichen Faserverlauf zeigen und durch die prozessinhärente Anpressung der Bandkuchenlagen an den Bandablageoberteil für die Messung konditioniert werden.

Die Fig. 5a und 5b zeigen eine andere prozesseigene Ablageweise der Faserbänder, bei welcher der Bandwickel um ein erstes und zweites Drehzentrum so abgelegt wird, dass die Faserbänder spiralförmig um ein Zentrum angeordnet werden. Auch hier findet eine Anpressung der Faserbänder durch den Kannenboden gegen den Bandablageoberteil statt.

Im Bandablageoberteil 21 ist, wie in Fig. 4 schon gezeigt, die Messöffnung W angeordnet, an welche die oberste Faserbandlage B angepresst und vorbeibewegt wird (siehe Fig. 5b). Wie eingangs schon erwähnt, kann die Messöffnung durch beispielsweise eine Planscheibe verschlossen oder aber offen sein (wobei dann die bandseitigen Lochränder entsprechend abgerundet sind). Solch eine Messöffnung ist, wenn kreisförmig, ca. 10 Millimeter im Durchmesser, so dass beide Möglichkeiten in Betracht gezogen werden können: die Minimierung von optischen Grenzflächen durch Weglassen einer Planscheibe oder eine Vergrösserung der Messöffnung, was in der Regel den Einsatz einer Planscheibe erfordert. Es können auch nichtplane optische Mittel, wie abbildende Linsen, eingesetzt werden. Solche mittel werden in der europäischen Patentanmeldung EP-A-0446808 beschrieben.

In radialer Richtung zur Drehachse der Kanne kann die Position der Messöffnung so ausgewählt werden, dass die Struktur des komprimierten, unter der Messöffnung vorbeilaufenden Fasergutes am feinsten ist, was in der Regel am Innenrand des gepressten Bandkuchens der Fall ist. Auf diese Weise lässt sich die Messstellen-Position optimieren.

Im Normalbetrieb werden in zeitlichem Abstand von z.B. 10 Sekunden Farbwerte des Kardenbandes gemessen. Der Kannenwechsel unterbricht die Farbmessungen für ca. 5-10% der Zeit, die zum Füllen einer Kanne benötigt wird. Dies ist eine Blindphase im Messablauf bzw. eine Messlücke. Diese Messlücke, in der keine Farbmessdaten gewonnen werden können, kann verringert bis aufgehoben werden, indem auf weiteren Kannenablagen parallel laufender Karden desselben Sortiments weitere Farbsensoren eingesetzt werden. Wird der Kannenwechsel zeitlich so organisiert, dass während des Wechsels von mindestens einem der Sensoren stets Farbdaten gewonnen werden, so ist es möglich, quasikontinuierlich die Farbe des Fasergutes zu messen. Dass alle Kannen miteinander gewechselt werden, ist praktisch auszuschliessen, insbesondere, wenn noch organisatorische Massnahmen bezgl. Zeit des Kannenwechsels dazukommen.

Schon mit einem zweiten Sensor an einer benachbarten Bandablage stört der Kannenwechsel zeitlich kaum mehr. Die Wahrscheinlichkeit, dass beide Bandablagen ihre Kannen gleichzeitig wechseln, ist vernachlässigbar gering. Eine zweite Messstelle (oder mehrere) hat ausserdem weitere Vorteile, nämlich Redundanz durch doppelt soviele Messungen, wenn gleichzeitig gemessen wird (lediglich während der kurzen Zeit des Kannenwechsels fällt die Redundanz dahin).

Die durch die Messung der genannten Konzentration gewonnenen Signale können in einer Steuerung derart ausgewertet werden, dass damit entweder der Anteil an z.B. Kunstfasern im ganzen Faserverband an einem Bildschirm (nicht gezeigt) angezeigt oder in einer in der EP-A-0 362 538 verschriebenen Steuerung zur Beeinflussung der Mischung verwendet werden.

Besonderer Vorteil der Redundanz:
- gegenseitige Überprüfung der Sensoren durch Vergleich der Signale,
- Feststellen von ev. Entmischungs-Vorgängen im Kardenschacht und in der Karde.

## Patentansprüche

1. Verfahren zum Prüfen einer aus verschiedenen Faserarten gebildeten Mischung,wobei aus der Mischung ein Faserverband gebildet wird, dadurch gekennzeichnet, dass der Faserverband als Gesamtgemisch einem Messverfahren unterworfen wird, womit durch Auswertung von Spektren im nahen Infrarotbereich die Konzentration einzelner vorgegebener Faserarten im Faserverband feststellen lässt und dass die durch diese Messung gewonnenen Signale in einer Steuerung ausgewertet werden, um den Anteil mindestens einer Faserart im Gesamtgemisch festzustellen.

2. Verfahren nach Anspruch 1, dadurch gekennzeichnet, dass die Messung am laufenden Faserverband durchgeführt wird.

3. Verfahren nach Anspruch 1 oder 2, dadurch gekennzeichnet, dass die Messung an einer Stelle geschieht, an welcher eine Verdichtung des Faserverbandes durch Zusammenführen oder Pressung stattfindet.

4. Verrfahren nach einem der Ansprüche 1 bis 3, dadurch gekennzeichnet, dass die Messung durch Beleuchten des Faserverbandes stattfindet, wobei an der Messstelle eine Lichtdurchführung so angeordnet wird, dass der Prozessablauf nicht beeinträchtigt wird, und durch diese Lichtdurchführung das vom Messgut senkrecht zur Längsachse des Faserverbandes reflektierte Licht aufgefangen und ausgewertet wird.

5. Verfahren nach Anspruch 4, dadurch gekennzeichnet, dass als Messstelle eine Stelle gewählt wird, an der der Faserverband in sich überlagernden Schichten abgelegt ist.

6. Verfahren nach einem der Ansprüche 1 bis 5, dadurch gekennzeichnet, dass die Mischung aus Mischkomponenten gebildet ist, die steuerbar variabel sind, und das Steuern des Mischverfahrens anhand der von der Steuerung ausgewerteten Signale erfolgt.

7. Verfahren nach einem der Ansprüche 1 bis 6, dadurch gekennzeichnet, dass die Mischung sowohl natürliche wie auch künstlich hergestellte Fasern umfasst und den Anteil der Kunsffasern festgestellt wird.

8. Verfahren nach einem der Ansprüche 1 bis 7, dadurch gekennzeichnet, dass die Prozessstelle derart gewählt ist, dass die Messung an einem Kardenband erfolgt.

9. Vorrichtung zum Prüfen einer aus verschiedenen Faserarten gebildeten Mischung, die in der Form eines Faserverbandes vorliegt, dadurch gekennzeichnet, dass ein Messgerät in der Form eines Infrarot-Spektrometers und eine Steuerung vorgesehen sind, wobei dem Messgerät der Faserverband als Gesamtgemisch zugeführt werden kann und das Gerät dazu geeignet ist, die Konzentration einzelner vorgegebener Faserarten im Faserverband festzustellen und durch diese Messung gewonnene Signale an die Steuerung zu leiten, und wobei die Steuerung diese Signale auswerten kann, um den Anteil mindestens einer Faserart im Gesamtgemisch festzustellen.

10. Vorrichtung nach Anspruch 9, dadurch gekennzeichnet, dass ein Mittel vorgesehen ist, welches das Fasergut beim Zuführen an die Messstelle komprimiert.

11. Vorrichtung nach Anspruch 9 oder 10, dadurch gekennzeichnet, dass die Messstelle einen Bandtrichter (22) und eine senkrecht zum Trichterhals (23) angeordnete Messonde umfasst und dass der Bandtrichter (23) mit einer Durchgangsöffnung versehen ist, deren Querschnitt die Form eines flachen Rechteckes aufweist, und dessen der Messonde zugewandte Wand (24) durchsichtig ist, während die gegenüberliegende Wand (25) keine besondere Eigenschaften in bezug auf das Messen aufweist.

12. Vorrichtung nach einem der Ansprüche 9 bis 11, dadurch gekennzeichnet, dass die Vorrichtung beidseits des Bandes mindestens je einen Bandtransportriemen (30,31,32) auf der einen Seite des Bandes eine durchsichtige Platte (34) und eine auf der vom Faserverband abgewandten Seite der durchsichtigen Platte angebrachte Messonde (1) umfasst und, dass die Transportriemen und die durchsichtige Platte derart angeordnet sind, dass sich der Abstand zwischen ihnen, in Bandlaufrichtung gesehen, gegen die durchsichtige Platte sukzessive verkleinert und in der Gegend der durchsichtigen Platte ein Minimum annimmt.

13. Vorrichtung nach Anspruch 12, dadurch gekennzeichnet, dass auf der Messeite des Faserverbandes die durchsichtige Platte (34) zwischen zwei Transportriemen, einem Eingangs- (30) und einem Ausgangsriemen (31) angeordnet ist, und dass die Riemen (30 und 31) über plattenförmige Kulissen (36.1/2) unmittelbar an die durchsichtige Platte (34) herangeführt werden.

## Claims

1. A method for examining a mixture formed from different types of fibres, with a fibre bundle being formed from the mixture, characterized in that the fibre bundle is subjected to a measuring process as entire mixture, by means of which the concentration of individual predetermined types of fibres in the fibre bundle can be determined by evaluation of spectra in the near infrared range and that the signals gained by this measurement are evaluated in a control unit in order to determine the share of at least one type of fibre in the entire mixture.

2. A method as claimed in claim 1, characterized in that the measurement is carried out on the running fibre bundle.

3. A method as claimed in claim 1 or 2, characterized in that the measurement occurs at a position where a compacting of the fibre bundle occurs by funnelling or pressing.

4. A method as claimed in one of the claims 1 to 3, characterized in that the measurement occurs by illumination of the fibre bundle, with a light pass-through means being arranged at the measuring position in such a way that process procedure is not impaired, and that as a result of this light pass-through the light reflected by the measured material vertical to the longitudinal axis of the fibre bundle is caught and evaluated.

5. A method as claimed in claim 4, characterized in that a position is selected as measuring position where the fibre bundle is deposited in mutually overlapping layers.

6. A method as claimed in one of the claims 1 to 5, characterized in that the mixture is formed of mixing components which are variable in a controlled manner and that the control of the mixing process occurs on the basis of the signals evaluated by the control unit.

7. A method as claimed in one of the claims 1 to 6, characterized in that the mixture comprises both natural as well as artificially produced fibres and the share of artificial fibres is determined.

8. A method as claimed in one of the claims 1 to 7, characterized in that the process position is selected in such a way that the measurement occurs on a card sliver.

9. An apparatus for examining a mixture formed from different types of fibres which are present in the form of a fibre bundle, characterized in that a measuring device in form of an infrared spectrometer and a control unit are provided, whereby the fibre bundle can be supplied to the measuring device as a complete mixture and the device is capable of determining the concentration of individually predetermined types of fibres in the fibre bundle and of supplying the signals gained from this measurement to the control unit, and whereby the control unit can evaluate these signals in order to determine the share of at least one type of fibre in the total mixture.

10. An apparatus as claimed in claim 9, characterized in that a means is provided which compresses the fibre material during the supply to the measuring position.

11. An apparatus as claimed in claim 9 or 10, characterized in that the measuring position comprises a sliver funnel (22) and a measuring probe arranged vertical to the funnel throat (23) and that the sliver funnel (23) is provided with a pass-through opening whose cross section has the shape of a flat rectangle and whose wall (24) facing the measuring probe is transparent, whereas the opposite wall (25) is not provided with any specific properties in respect of the measuring.

12. An apparatus as claimed in one of the claims 9 to 11, characterized in that the apparatus comprises at either side of the sliver at least one sliver conveyor belt (30, 31, 32), a transparent plate (34) on one side of the sliver and a measuring probe (1) attached on the side of the fibre bundle which is averted from the transparent plate, and that the conveyor belts and the transparent plate are arranged in such a way that the distance between them, as seen in the running direction of the sliver, successively decreases towards the transparent plate and assumes a minimum in the vicinity of the transparent plate.

13. An apparatus as claimed in claim 12, characterized in that on the measuring side of the fibre bundle the transparent plate (34) is arranged between two conveyor belts, an input belt (30) and an output belt (31), and that the belts (30 and 31) are guided directly towards the transparent plate (34) by means of plate-like connecting links (36.1/2).

## Revendications

1. Procédé servant à examiner un mélange formé de différents types de fibres, et où un assemblage de fibres est formé à partir du mélange,
caractérisé par le fait que
l'assemblage de fibres, en tant que mélange total, est soumis à un procédé de mesure, à l'aide duquel la concentration des types individuels de fibres, prédéterminés dans l'assemblage de fibres, peut être détectée par analyse de spectres dans la zone proche des infrarouges, et que les signaux obtenus par cette mesure sont évalués dans un asservissement, afin de déterminer la quantité d'au moins un type de fibres dans le mélange total.

2. Procédé selon revendication 1,
caractérisé par le fait que
la mesure est exécutée pendant le déplacement de l'assemblage de fibres.

3. Procédé selon revendication 1 ou 2,
caractérisé par le fait que
la mesure est exécutée à un endroit où une condensation de l'assemblage de fibres est réalisée par rassemblement ou compression.

4. Procédé selon une des revendications 1 à 3,
caractérisé par le fait que
la mesure est réalisée par illumination de l'assemblage de fibres, et où, au lieu de mesure, un guidage de lumière est agencé de telle sorte que le processus de déroulement n'est pas entravé, et, par ce guidage de lumière, la lumière réfléchie par la matière à mesurer est interceptée perpendiculairement à l'axe longitudinal de l'assemblage de fibres, et est évaluée.

5. Procédé selon revendication 4,
caractérisé par le fait que,
comme lieu de mesure, un endroit est choisi dans lequel l'assemblage de fibres est déposé en couches qui se superposent.

6. Procédé selon une des revendications 1 à 5,
caractérisé par le fait que
le mélange est constitué par des composants de mélange qui sont variables d'une manière commandable, et la commande du procédé de mélange est réalisée en fonction des signaux évalués par l'asservissement.

7. Procédé selon une des revendications 1 à 6,
caractérisé par le fait que
le mélange comprend aussi bien des fibres naturelles que des fibres fabriquées d'une manière artificielle, et la quantité des fibres artificielles est détectée.

8. Procédé selon une des revendications 1 à 7,
caractérisé par le fait que
le lieu de processus est choisi de telle manière que la mesure est exécutée sur un ruban de carde.

9. Dispositif servant à examiner un mélange formé de différents types de fibres, lequel se présente sous forme d'un assemblage de fibres,
caractérisé par le fait
qu'un appareil de mesure sous forme d'un spectromètre infrarouge et un asservissement sont prévus, et où l'assemblage de fibres peut être guidé vers l'appareil de mesure en tant que mélange total, et l'appareil est approprié pour déterminer la concentration des types individuels de fibres, prédéterminés dans l'assemblage de fibres, et pour guider vers l'asservissement des signaux obtenus par cette mesure, et où l'asservissement peut évaluer ces signaux, afin de déterminer la quantité d'au moins un type de fibres dans le mélange total.

10. Dispositif selon revendication 9,
caractérisé par le fait
qu'un moyen est prévu qui comprime la matière fibreuse lors de son amenée dans le lieu de mesure.

11. Dispositif selon revendication 9 ou 10,
caractérisé par le fait que
le lieu de mesure comprend un entonnoir de ruban (22) et une sonde de mesure disposée perpendiculairement au col d'entonnoir (23), et que le col d'entonnoir (23) est pourvu d'une ouverture de passage dont la section présente la forme d'un rectangle applati, et dont la paroi (24) faisant face à la sonde de mesure, est transparente, alors que la paroi opposée (25) ne possède aucune caractéristique particulière concernant la mesure.

12. Dispositif selon une des revendications 9 à 11,
caractérisé par le fait que
le dispositif comprend, des deux côtés de l'assemblage de fibres, au moins une courroie de bande transporteuse (30,31,32), et, sur un côté de l'assemblage de fibres, il comprend une plaque transparente (34) et une sonde de mesure (1) disposée du côté opposé au côté de la plaque transparante faisant face à l'assemblage de fibres, et que les courroies de bande transporteuse et la plaque transparente sont disposées de telle manière que, vu dans le sens de déplacement des bandes, la distance entre celles-ci diminue successivement en allant vers la plaque transparente, et possède un minimum dans les environs de la plaque transparente.

13. Dispositif selon revendication 12,
caractérisé par le fait que,
du côté de la mesure de l'assemblage de fibres, la plaque transparente (34) est diposée entre deux courroies de bande transporteuse, dont une d'entrée (30) et une de sortie (31), et que les courroies (30 et 31) sont guidées immédiatement près de la plaque transparente (34) à l'aide de coulisses (36.1/2) en forme de plaques.
